# EUROPEAN PATENT SPECIFICATION

(11) **EP 2 563 281 B1**
(45) Date of publication and mention of the grant of the patent: **26.09.2018**
(21) Application number: 11718150.3
(22) Date of filing: 28.04.2011
(51) Int. Cl.: A61F 2/34, A61F 2/30, A61F 2/00

(54) **PROSTHESIS**
PROTHESE
PROTHÈSE

(30) Priority: 29.04.2010 GB 201007166
(43) Date of publication of application: 06.03.2013
(73) Proprietor: Finsbury (Development) Limited, Leeds, West Yorkshire LS11 8DT (GB)
(72) Inventor: TAYLOR, Andrew Clive, Nr. Chicester Sussex PO19 3QQ (GB)
(74) Representative: Alton, Andrew
(86) International application number: PCT/GB2011/050853
(87) International publication number: WO 2011/135377

(56) References cited:
- EP-A1- 0 013 863
- EP-A1- 0 552 950
- EP-A1- 0 668 062
- EP-A1- 1 527 757
- EP-A2- 1 854 430
- WO-A1-97/38649
- WO-A1-2006/125711
- WO-A2-2009/034429
- DE-A1- 3 416 471
- GB-A- 2 268 408
- KR-A- 20080 093 846
- US-A- 5 163 961
- US-A- 5 549 691
- US-A1- 2009 192 610

## Description

The present invention relates to an acetabular prosthesis.

The efficient functioning of the hip joint is extremely important to the well-being and mobility of the human body. Each hip joint is comprised by the upper portion of the femur which terminates in an offset bony neck surmounted by a ball-headed portion which rotates within the acetabulum in the pelvis. Diseases such as rheumatoid- and osteo-arthritis can cause erosion of the cartilage lining of the acetabulum so that the ball of the femur and the hip bone rub together causing pain and further erosion. Bone erosion may cause the bones themselves to attempt to compensate for the erosion which may result in the bone becoming misshapen.

Operations to replace the hip joint with an artificial implant are well-known and widely practiced. Generally, the hip prosthesis will be formed of two components, namely: an acetabular component which lines the acetabulum; and a femoral component which replaces the femoral head. The femoral component may be total femoral head replacement in which case the component includes a head, neck and a stem which in use is inserted into the end of a prepared femur. Alternatively, where appropriate, the femoral head component may be a resurfacing prosthesis which is attached to the head of the femur once it has been suitably machined.

In an operation to insert a prosthetic acetabulum in a patient's pelvis the surgeon first uses a reamer to cut a cavity of appropriate size in the patient's pelvis. An acetabular cup is then inserted into the cavity. By "appropriate size" is meant a size which is selected by the surgeon as being the most appropriate for that particular patient. Normally, it is desirable to retain as much of the original healthy bone surface as possible.

Commercially available acetabular cups are sold in a range of sizes to suit the needs of individual patients. Generally, acetabular cups are available in sizes of from 42 mm to 62 mm diameter with 2 mm increments between neighboring sizes.

There are a number of different types of prosthetic acetabular cups. One type of cup is that made from polyethylene. They are generally cemented into the acetabulum and require only light pressure to seat them in the cement.

One alternative cup type has a polyethylene liner unit for articulation with the femur and a metal shell for insertion into the pelvic cavity. These cups with metal shells may be implanted without cement such that they rely on a jam fit between the metal shell and the patient's acetabulum. However, in some arrangements, screws may be used to secure the cup shell in position in the pelvis before the liner is applied into position. The insertion of the metal shell into the pelvis requires considerable force. As the surgeon applies this force, there is a risk that the metal shell can become damaged or deformed. There is also a possibility that during the application of the force, the shell may be moved so that it is not in the optimum alignment in the acetabulum. Often the metal shells have outer surfaces or coatings which encourage bone to grow into them over time.

With this type of prosthesis, the polyethylene liner unit is snapped or screwed into the metal shell after the metal shell has been seated in the acetabulum. Thus the inner surface of the liner forms the socket part of the joint.

More recently, ceramic liners have been used as an alternative to the plastics liner. In this arrangement, the metal shell, which is generally formed from titanium, is first inserted into the acetabulum. The ceramic liner is then inserted into the shell.

Whilst these various prior art arrangements offer relief to patients from the pain of the worn joint, there is a continuing desire to provide prostheses which provide improved results to the patient, particularly in terms of wear. This is particularly important for younger patients where revision operations may be required if the prosthesis itself becomes worn or becomes loosened in the acetabulum. There is therefore an ongoing need for new materials and/or new structures for prostheses which more closely mimic the natural bone while minimizing the wear problems thereof.

Polyetheretherketone (PEEK) polymers have been suggested as being suitable materials for use in orthopaedic implants. This material is discussed in "Taking a PEEK at Material Options for Orthopedics", Kinburn A., Medical Design Technology Magazine; 1 Jan 2009 page 26ff. It is suggested that particular advantages can be obtained if the PEEK is reinforced with carbon fibres. The use of PEEK in a horseshoe-shaped cup is discussed in "Biomechanics of a PEEK Horseshoe-Shaped Cup: Comparisons with a Predicate Deformable Cup" Manley, M. T., et al Poster No 1717 at 53rd Annual Meeting of the Orthopaedic Research Society.

WO2009/097412 suggests that PEEK can be useful in a multi-layered hemispherical prosthesis. The multi-layered system comprises an outer layer formed from a porous metal and an inner layer formed from a polyaryletherketone in which the polyaryletherketone at least partially permeates the pores of the first material. The Applicants suggest that this arrangement allows the two layers to be securely bonded together. They also suggest that the prosthesis has a lower stiffness than that achieved for prior art arrangements.

In an application filed by Finsbury (Development) Limited on 3^{th} February 2011 and accorded application number PCT/GB2011/050188, published as WO-2011/095813 there is described an improved prosthesis comprising:
an inner layer formed from a polyaryletherketone;
a first outer layer adjacent to said inner layer formed from a porous polyaryletherketone at least some of said pores having located therein material to promote osteointegration; and
a second outer layer adjacent to said first outer layer formed from a porous polyaryletherketone, a portion of said pores being free of material to promote osteointegration.

A second embodiment described in PCT/GB2011/050188 relates to a prosthesis which comprises:
an inner layer formed from a polyaryletherketone;
a first outer layer adjacent to said inner layer formed from a porous polyaryletherketone, at least some of said pores having located therein material to promote osteointegration having a crystallinity of from about 60% to about 90%; and
a second outer layer adjacent to said first outer layer formed from a porous polyaryletherketone, at least a portion of said pores having located therein material to promote osteointegration having a crystallinity of less than about 50%.

These prostheses offer various advantages over prior art arrangements. The prosthesis is lightweight, while having the required strength. In addition, it has improved wear resistance over prior art arrangements.

In the arrangement of PCT/GB2011/050188.7 osteointegration is provided for by means of an outer layer having pores at least some of which are provided with material which promotes osteointegration.

Other approaches to enhance osteointegration have been suggested. For example in some arrangements, the bone-facing surface of the prosthesis is supplied with upstanding projections which allow for improved osteointegration. For example, in EP1527757, an element is described which comprises a rear face, a front face, and a body extending between the rear face and the front face, wherein the front face comprises a basal surface and at least one upstanding portion projecting above the basal surface and having a transverse hole extending therethrough from one side of the upstanding portion to another. These elements are incorporated into the bone-facing surface of the prosthesis. Examples of other osteointegration projections located on the surface of prosthesis are given in WO2009/034429, EP0668062, FR2548889 and EP0552950. In an alternative approach, it has been suggested that the surface of the prosthesis should be roughened or include grooves or channels. Examples of these arrangements can be found in GB2268408 and EP1854430.

Whilst these arrangements all assist osteointegration to occur, it does take some time for the bone to integrate with the prosthesis and hold it firmly in place. Thus when the prosthesis is first impacted into position, the hold later provided by osteointegration does not apply. There is therefore a need to provide a means for holding the prosthesis in position during the initial stages of use.

Whilst conventionally screws or similar mechanical fixation methods can be used to hold the prosthesis in position, these are generally undesirable. For example, in an acetabular cup prosthesis, the presence of screw head(s) is disadvantageous as it will interrupt the articulating surface or will require the use of a liner within the cup such that the screw head does not impinge on the articulating surface. There is therefore a need for an improved prosthesis which addresses the problem of initial fixation and which does not have the problems noted with conventional mechanical fixation means.

EP-A-0013863 discloses an acetabular prosthesis having a bone-facing surface from which a plurality of pins with a sawtooth-like profile extend. The pins are positioned within a bore within the bone. The bone can then grow into the notches provide along the pin to improve anchoring of the prosthesis within the bone.

According to the present invention there is a provided an acetabular cup prosthesis as defined in claim 1.

The prosthesis is provided with a plurality of barbed stakes. By "barbed" stake, we mean that the stake has at least one barb which whilst allowing the stake to be impacted into the bone make its extraction difficult. The barb may be a backward facing protrusion such as one would find on, for example, a fish-hook or arrow or may be a protrusion extending substantially perpendicularly from the stake. In one arrangement, the barb may extend around the stake. Whilst the, or each, barb may be located at any point on the stake, in a preferred arrangement it will generally be located on the distal end of the stake, i.e. the end remote from the surface of the prosthesis. In one arrangement however, further barbs may be provided on the stake closer to the surface of the prosthesis. Whilst in one arrangement, the distal end of the stake may be pointed to facilitate impaction into the bone, the surface may be of any suitable shape. In one arrangement it is curved.

The present invention enables the stakes to be driven into the bone as the prosthesis is impacted into position. The, or each, barb prevents the prosthesis from being withdrawn and therefore serves as an initial fixing means. The stakes will generally be angled such that they align with the impaction angle of the prosthesis. Where the prosthesis is an acetabular cup prosthesis, this angle does not align with the axis passing through the pole of the cup. The alignment will depend on the design of the particular cup but will generally be at about 10° to about 30°, preferably about 15° to about 25°, or about 20° from the axis passing through the pole of the cup. In addition to being in the impaction direction this will also be in the loading direction in use therefore improving stability.

For strength, the stakes and barbs will generally be formed as a single unit. Since the stakes are integral with the body of the prosthesis, they will generally be formed with the prosthesis. In a particularly preferred arrangement, the prosthesis is formed from a polymer and the stakes are formed integrally with the prosthesis as part of the moulding process. The barbs may be formed as part of the mould, or in one arrangement the stakes, once formed, may be contacted with a heated tool to form a barb arrangement at the distal end of the pre-formed stake. It will be understood that where the stake is formed from the thermoplastic polymer, the tool will be heated to a temperature above the melting point of the polymer. Heating of the tool may be achieved by any suitable means. In one arrangement this may be achieved by induction heating.

The stakes may be of any suitable size. Suitable sizes are generally of from about 0.3mm to about 3mm in length. The length of the stakes may be the same or different. The width of the shaft of the stake may be from about 0.2mm to about 3mm. The width of the stake at the widest point of the barb will be wider than that of the shaft of the stake and may be twice the width thereof. The shaft of the stake may be straight or in one arrangement it may taper away from the body of the prosthesis to the underside of the barb.

They stakes may be selected to be the same length from the surface of the bone-facing surface of the body of the prosthesis and/or they may be selected so that their distal ends align in a particular orientation as desired which may mean that the stakes are of differing length to achieve the desired configuration.

The stakes will generally be configured to have the required strength to withstand the impaction forces and to allow the stakes to be driven into the bone. Where the prosthesis is to be inserted into the acetabulum stakes must be able to be driven into the acetabulum post machining. The stiffness of the acetabulum post machining would be approximately that of metphysis or lower, and may approach cancellous. As indicated in Taylor, Tanner & Freeman, J. Biomechanics 31 (1998) 303 - 319, the Young's Modulus of diaphysis cortical bone is 17GPa, metaphysis cortical bone is 5Gpa and that of cancellous bone is 0.3 - 0.4 GPa.

Stakes may be provided over the whole of the bone-facing region of the prosthesis or over a portion thereof. Generally, from about 25% to substantially 100% of the bone-facing region of the prosthesis will have stakes extending therefrom. However, amounts of from about 30% to about 75% are preferred.

The stakes will generally be spaced from about 0.5mm to about 15mm, with spacings of 1mm or 2 mm to about 12 mm, and 3mm to about 10mm being useful. In one arrangement the stakes will be located at the main articulation area of the prosthesis i.e. in the region where most loading is applied to the prosthesis. Where the surface of the prosthesis is to be coated with a material to promote osteointegration such as hydroxyapatite, it is important that the spacing of the stakes is not so close that the gap between the stakes fills up with the bone ingrowth material. The osteointegration material will generally be applied at a thickness of about 0.3 mm but it can build up against external projections.

The spacing of the stakes may be the same throughout the surface carrying the stakes or in one other arrangement, they may be more closely spaced. For example, they may be more densly packed in an area carrying most load.

The stakes may be provided as discrete protrusions. They may be of any cross-section with circular or square being preferred.

The plurality of stakes are conjoined to provide a network arrangement. Where a network is present, more than one network may be present. The network may be of any suitable configuration. In one arrangement, the stake may be elongated such that it becomes a wall with a barb extending from each side thereof. The barb may be located at or near the top of said wall. The walls may be arranged in any suitable arrangement. In one arrangement, the walls may be arranged to in a triangular or hexagonal arrangement. Where walls meet, the region of intersection may be free of stake/wall or may have a lower height region which may or may not have a barb. The benefit of using walls is that the stakes are stronger and less likely to break during impaction.

The prosthesis may be formed of any suitable material. In one arrangement it is formed from a thermoplastic polymer. Any thermoplastic polymer may be used provided that it is biocompatible or is coated with biocompatible material. Suitable thermoplstics include a member of the family of polyaryletherketones with polyetheretherketone and polyetherketoneketone being particularly useful. In one arrangement, the thermoplastics polymer may be reinforced. The stakes will generally be formed with the prosthesis and will therefore generally be formed from the same material.

Where the prosthesis is formed from a thermoplastic polymer, it may be reinforced. Any suitable material may be used to reinforce the polymer such as the polyaryletherketone although carbon fibre is particularly preferred as the reinforcing material. Where the thermoplastic polymer is reinforced, it may be reinforced throughout the prosthesis such that reinforcing continues into the, or each, stake. Alternatively, reinforcement may only be provided in a portion of the prosthesis, for example in a region remote from the bone-facing surface.

In one arrangement, the reinforcement may be aligned to provide specific desired properties. For example, they may be aligned within each stake to maximise their strength.

The prosthesis may be formed of more than one layer. Where more than one layer is present a layer which will form the articulating surface may be reinforced and a layer forming the bone-facing surface may not be reinforced. In one arrangement, the prosthesis may comprise the dual layer configuration described in PCT/GB2011/050188.

Material to promote osteointegration may be coated on the bone-facing surface of the prosthesis and/or on the surface of the stakes and/or barbs. Any suitable material may be used including hydroxyapetite.

In an alternative arrangement of the present invention, the prosthesis may additionally include at least one locating rod extending from the bone-facing surface. In use this would be located in a well drilled in the bone by the surgeon and would assist to ensure that the prosthesis is located in the correct position before impaction occurs. The location of the well can be obtained using any suitable locating device or jig. The rod will generally be longer that the stake and/or may be of a larger cross-section. The rod may be located on any suitable place on the bone-facing surface of the prosthesis. In one arrangement, the rod may have at least one barb located thereon and/or may be pointed. In one arrangement locating rods may be present. In one arrangement three rods may be present. Where three are present they will generally be located in a triangular configuration as this gives the greatest stability. Generally they will be located such that in use they are directed in the appropriate orientation for the bone into which they are to be implanted. Where the prosthesis is an acetabular hip prosthesis, they will generally be orientated such that they impact into the thickest parts of the bone, i.e. into the ilium, the public bone and the uschium respectively.

The prosthesis is an acetabular prosthesis. In one arrangement it may be of a conventional cup shape. However, since in the natural pelvis the acetabulum is not a hemisphere and is more akin to a horseshoe shape, the prosthesis may be of a similar horseshoe shape. It is known that even when a cup shaped prosthesis is used, the wear on the cup by the femoral head prosthesis subscribes a horseshoe.

In one alternative arrangement the prosthesis may be of a combination structure whereby the cup is overall of conventional hemispherical cup but is thicker in the bearing region, i.e. in the horseshoe, and thinner in the remaining area. This thinner area may be the location of fixing means to the bone. Thus where bone screws are to be used, they may be inserted through the prosthesis at this position. Where a combination structure is used, the outer surface may generally be a conventional hemispherical cup and the increased thickness in the bearing region will lead to a raised bearing surface in the inner surface of the cup.

The prosthesis of the present invention when of a conventional hemispherical structure will be of any suitable size and the walls of any suitable thickness. The thickness will generally be the minimum required to conserve bone and maintain as much of the natural femur head diameter as possible. In one arrangement, the wall thickness will be from about 2 to about 5 mm with about 3 mm being generally useful. The ratio of the thickness of the inner layer to the outer layer may be adjusted as required. However, it is believed that a ratio in the region of 1:1 may be desirable. However a variation in the thickness ratio allows the properties of the prosthesis to be tailored such that the natural distribution of loads into the pelvis is replicated.

Where the prosthesis is of the arrangement where there is an increased thickness in the load bearing area and a thinner area in non-load bearing positions, the thickness of the load bearing area will be similar to that discussed above for conventional prosthesis. In one arrangement, an aperture may be located in the non-load bearing position. This can be useful to assist with introduction of the prosthesis and allows lubrication to flow through the prosthesis.

The present invention will now be described, by way of example, with reference to the accompanying drawings in which:
- Figure 1: is a perspective view from below of an acetabular cup prosthesis prior to the formation of the barbs on the stakes;
- Figure 2: is a perspective view of the acetabular cup prosthesis of Figure 1 after the barbs have been formed;
- Figure 3: is a schematic simplified illustration of one method by which the barb may be formed with Figure 3a illustrating the barb before stake formation and Figure 3b illustrating the tool in position forming the barb;
- Figure 4: is a perspective view from one side and below of an acetabular cup prosthesis of the present invention wherein the stakes are conjoined to form a network:
- Figure 5: is a partial cut-through view of the prosthesis of Figure 4 from below;
- Figure 6: is a blown up view of a portion of Figure 5;
- Figure 7: is a partial cut-through view of the prosthesis of Figure 4 from the side;
- Figure 8: is a perspective view of an alternative embodiment of the present invention from below;
- Figure 9: is a schematic illustration of the acetabular cup prosthesis of Figure 7 in position in the pelvis; and
- Figure 10: is a view of an acetabular prosthesis of one arrangement from above.

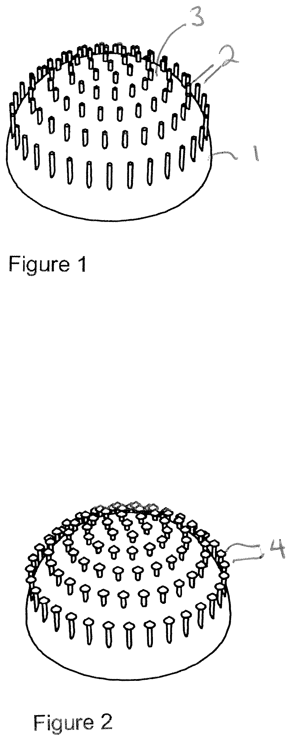

As illustrated in Figure 1, the acetabular prosthesis 1 the external bone-facing surface of the prosthesis is provided with a plurality of stakes 2 extending from the surface 3. In the illustrated arrangement, stakes 2 are spaced over the entirety of the outer surface 3.

In all of the Figures filed herewith, the size of the stakes and barbs has been exaggerated to improve the clarity of the illustration.

In the arrangement illustrated, the stakes 3 have been formed as part of the injection moulding process for the acetabular cup prosthesis which is formed from polyetheretherketone and as such are integral with the prosthesis 1. Once the stakes have been formed, they are pressed into a hot tool such that they splay to form the barbs 4 as shown in Figure 2.

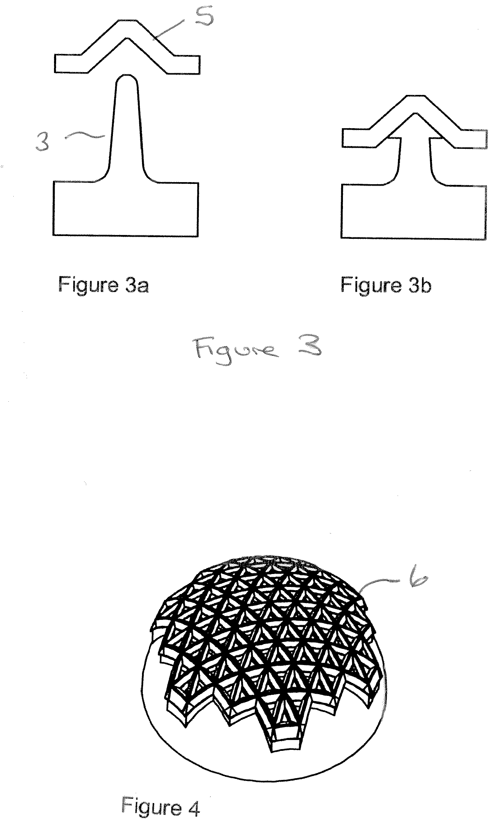

The process of barb 4 formation is illustrated schematically in Figures 3a and 3b. In the illustrated arrangement the stake is tapers from the base towards the point thereof but it will be understood that it may be of any suitable arrangement. As the stake is contacted with the heated tool 5 under force, it is deformed such that a portion of the stake 5 splays out to form a barb 4 which extends along both sides of the stake. Where the stake is of circular cross-section, the tool may be configured to form a barb surrounding the stake. In the illustrated embodiment the barb extends substantially perpendicularly from the stake as illustrated in Figure 3b.

An embodiment of the invention in which the stakes 3 are conjoined to form a network 6 is illustrated in Figure 4. This network can extend over substantially the whole area of the outer surface of the acetabular cup prosthesis or may be in one or more regions thereof. These regions may be selected to ensure that in use the prosthesis is firmly anchored and therefore are positioned to resist movement occasioned by the articulation of the femoral head or prosthesis therefor.

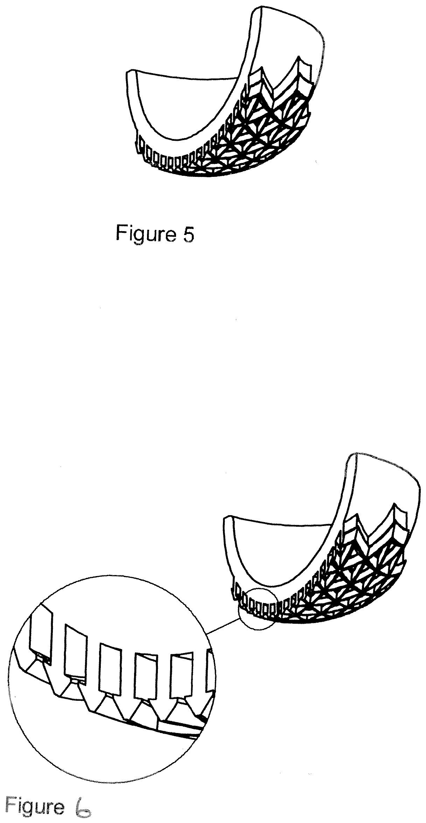

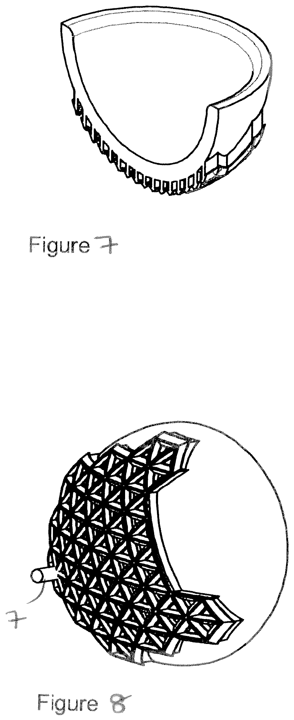

The shaping of the barbs in this network arrangement can be seen clearly in Figures 6 and 7.

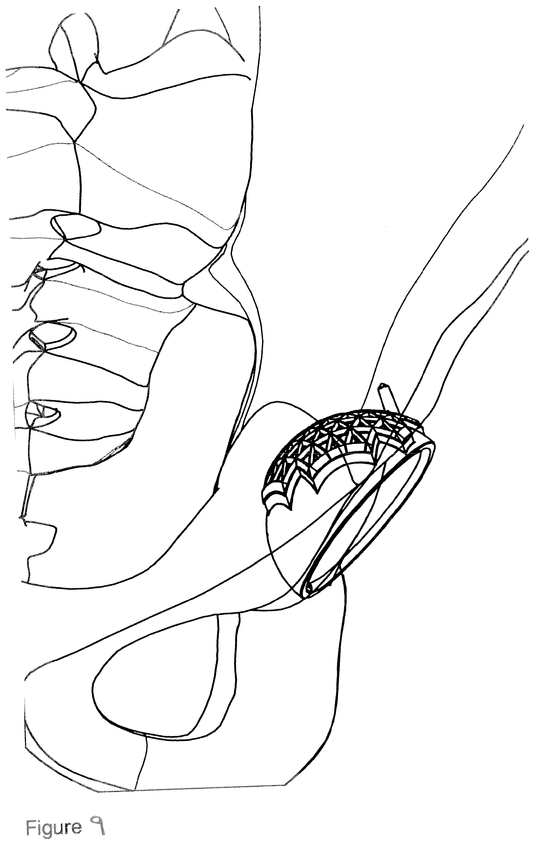

As illustrated in Figure 8 an alignment rod 7 may be provided to assist in the location of the prosthesis in the pelvis. The prosthesis in position is illustrated in Figure 9. For clarity only a portion of the network of stakes is illustrated and the pelvis is shown as translucent. Whilst the rim of the cup is illustrated as extending above the surface of the pelvis, the cup may be configured so that this does not occur.

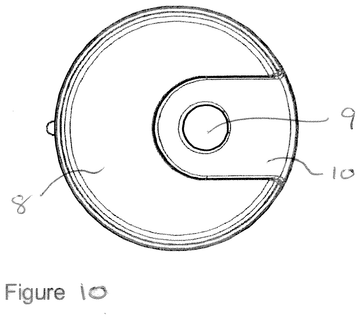

The embodiment of the invention where the prosthesis has a thicker region in a horse shoe configuration is illustrated in Figure 10. The thicker region 8 provides the articulating surface. An aperture 9 is provided in the thinner region 10.

Whilst the present invention has been discussed in detail in connection with an acetabular prosthesis, it will be understood that the invention may be applied to other prosthesis such as prosthesis in the shoulder. Further, it will be understood that various modifications, uses or adaptations of the invention may be made within the scope of this invention.

## Claims

1. An acetabular cup prosthesis (1) for fixation to a bone, the acetabular cup prosthesis comprising a plastics body and a plurality of barbed stakes (2) extending from a bone-facing surface (3) of said body; said plurality of barbed stakes being sized and located so as to provide fixation to the bone when the bone-facing surface is impacted into the bone, **characterized in that** the plurality of barbed stakes are conjoined to form walls, the walls being arranged to meet at intersections to form a network.

2. An acetabular cup prosthesis (1) according to Claim 1 wherein the barb (4) on each of the plurality of barbed stakes extends around the stake (2).

3. An acetabular cup prosthesis (1) according to Claim 1 or 2 wherein each of the plurality of barbed stakes (2) is from about 0.3 mm to about 3mm in length.

4. An acetabular cup prosthesis (1) according to any one of Claims 1 to 3 wherein the prosthesis has a pole, and wherein each of the plurality of barbed stakes (2) is angled between about 10° and 30° from an axis passing through the pole.

5. An acetabular cup prosthesis (1) according to Claim 1 wherein each of the plurality of barbed the stakes is spaced from about 0.5 mm to about 15 mm.

6. An acetabular cup prosthesis (1) according to any one of Claims 1 to 5 wherein the prosthesis is formed from a thermoplastic polymer.

7. An acetabular cup prosthesis (1) according to Claim 6 wherein the thermoplastic material is polyetheretherketone or polyetherketoneketone which may be reinforced.

8. An acetabular cup prosthesis (1) according to any one of Claims 1 to 7 wherein the prosthesis additionally comprises a locating rod (7) extending from the bone-facing surface (3).

9. An acetabular cup prosthesis (1) according to Claim 8 wherein each of the plurality of barbed stakes (2) extends parallel to the locating rod (7).

## Patentansprüche

1. Hüftgelenkpfannenprothese (1) für die Fixierung an einem Knochen, wobei die Hüftgelenkpfannenprothese einen Kunststoffkörper und ein Vielzahl von Stachelpflöcken (2) aufweist, die sich von einer knochenzugewandten Fläche (3) des Körpers erstrecken; wobei die Vielzahl von Stachelpflöcken derart bemessen und angeordnet ist, dass sie eine Fixierung an dem Knochen bereitstellen, wenn die knochenzugewandten Fläche in den Knochen auftrifft, **dadurch gekennzeichnet, dass** die Vielzahl von Stachelpflöcken verbunden sind, um Wände zu bilden, wobei die Wände derart angeordnet sind, dass sie sich an Schnittpunkten treffen, um ein Netzwerk zu bilden.

2. Hüftgelenkpfannenprothese (1) nach Anspruch 1, wobei der Stachel (4) jedem der Vielzahl von Stachelpflöcken sich um den Pflock (2) herum erstreckt.

3. Hüftgelenkpfannenprothese (1) nach Anspruch 1 oder 2, wobei jeder der Vielzahl von Stachelpflöcken (2) etwa 0,3 mm bis etwa 3 mm Länge hat.

4. Hüftgelenkpfannenprothese (1) nach einem der Ansprüche 1 bis 3, wobei die Prothese einen Pfosten hat, und wobei jeder der Vielzahl von Stachelpflöcken (2) zwischen etwa 10° und 30° gegen eine Achse gewinkelt ist, die durch den Pfosten geht.

5. Hüftgelenkpfannenprothese (1) nach Anspruch 1, wobei jeder der Vielzahl von Stachelblöcken von etwa 0,5 mm bis etwa 15 mm beabstandet ist.

6. Hüftgelenkpfannenprothese (1) nach einem der Ansprüche 1 bis 5, wobei die Prothese aus einem thermoplastischen Polymer ausgebildet ist.

7. Hüftgelenkpfannenprothese (1) nach Anspruch 6, wobei das thermoplastische Material Polyetheretherketon oder Polyetherketonketon ist, der verstärkt werden kann.

8. Hüftgelenkpfannenprothese (1) nach einem der Ansprüche 1 bis 7, wobei die Prothese zusätzlich eine Führungsstange (7) aufweist, die sich von der knochenzugewandten Fläche (3) erstreckt.

9. Hüftgelenkpfannenprothese (1) nach Anspruch 8, wobei jeder der Vielzahl von Stachelblöcken (2) sich parallel zu der Führungsstange (7) erstreckt.

## Revendications

1. Prothèse de cupule acétabulaire (1) destinée à être fixée sur un os, la prothèse de cupule acétabulaire comprenant un corps en plastique et une pluralité de tiges à ardillon (2) s'étendant à partir d'une surface faisant face à l'os (3) dudit corps ; ladite pluralité de tiges à ardillon étant dimensionnées et positionnées afin de fournir la fixation à l'os lorsque la surface faisant face à l'os est impactée dans l'os, **caractérisée en ce que** la pluralité de tiges à ardillon sont réunies afin de former des parois, les parois étant agencées pour se rencontrer aux intersections afin de former un réseau.

2. Prothèse de cupule acétabulaire (1) selon la revendication 1, dans laquelle l'ardillon (4) sur chacune de la pluralité de tiges à ardillon s'étend autour de la tige (2).

3. Prothèse de cupule acétabulaire (1) selon la revendication 1 ou 2, dans laquelle chacune de la pluralité de tiges à ardillon (2) a une longueur allant d'environ 0,3 mm à environ 3 mm.

4. Prothèse de cupule acétabulaire (1) selon l'une quelconque des revendications 1 à 3, dans laquelle la prothèse a un pieu, et dans lequel chacune de la pluralité de tiges à ardillon (2) est coudée entre environ 10° et 30° à partir d'un axe passant par le pieu.

5. Prothèse de cupule acétabulaire (1) selon la revendication 1, dans laquelle chacune de la pluralité de tiges à ardillon est espacée d'environ 0,5 mm à environ 15 mm.

6. Prothèse de cupule acétabulaire (1) selon l'une quelconque des revendications 1 à 5, dans laquelle la prothèse est formée à partir d'un polymère thermoplastique.

7. Prothèse de cupule acétabulaire (1) selon la revendication 6, dans laquelle le matériau thermoplastique est un polyétheréthercétone ou un polyéthercétonecétone qui peut être renforcé.

8. Prothèse de cupule acétabulaire (1) selon l'une quelconque des revendications 1 à 7, dans laquelle la prothèse comprend, de plus, une tige de positionnement (7) s'étendant à partir de la surface faisant face à l'os (3).

9. Prothèse de cupule acétabulaire (1) selon la revendication 8, dans laquelle chacune de la pluralité de tiges à ardillon (2) s'étend parallèlement à la tige de positionnement (7).
